# EUROPEAN PATENT SPECIFICATION

(11) **EP 1 399 201 B1**
(45) Date of publication and mention of the grant of the patent: **11.04.2012**
(21) Application number: 02715685.0
(22) Date of filing: 11.01.2002
(51) Int. Cl.: A61B 1/00, A61M 1/00

(54) **DEVICE FOR IN-VIVO PROCEDURES**
VORRICHTUNG FÜR IN-VIVO-VERFAHREN
DISPOSITIF POUR TECHNIQUES IN VIVO

(30) Priority: 11.01.2001 US 260645 P; 11.01.2001 US 260646 P; 23.07.2001 US 307040 P; 15.08.2001 US 312081 P
(43) Date of publication of application: 24.03.2004
(73) Proprietor: Given Imaging Ltd., 20692 Yoqneam (IL)
(72) Inventor: GILREATH, Mark, Charlotte, NC 28277 (US); ASHERY, Yoram, 54421 Givat Shmuel (IL); MERON, Gavriel, 49556 Petach Tikva (IL)
(74) Representative: Dr. Graf & Partner AG
(86) International application number: PCT/IL2002/000026
(87) International publication number: WO 2002/055126

(56) References cited:
- US-A- 5 035 231
- US-A- 5 188 596
- US-A- 5 368 015
- US-A- 5 402 769
- US-A- 5 489 256
- US-A- 5 584 796
- US-A- 5 653 677
- US-A- 5 908 294
- US-A- 6 059 719
- US-A- 6 059 719
- US-A- 6 086 528
- US-A- 6 141 037

## Description

### FIELD OF THE INVENTION

The present invention relates to the field of medical devices. More specifically the invention relates to essentially self contained devices, for performing in-vivo procedures, inter alia, in emergency situations.

### BACKGROUND OF THE INVENTION

Medical procedures in body lumens and cavities, such as gastroenterology procedures and laparoscopic surgery procedures, may require specifically designed medical devices. Typically, the devices include a performing end (distal end) functionally coupled to a controlling end (proximal end). The performing end, which is inserted in to the body, is operated and manipulated by the controlling end, which is accessible to an external operator.

In some cases the device further includes a viewing or imaging element for simultaneously viewing and performing a procedure in vivo. In that case the device may be connected to a cable that connects the viewing or imaging element to an external power supply system, a light source and a processing unit. Such a device is disclosed in US 5,584,796.

A common device for in-vivo procedures, which includes an imager, is the endoscope. Endoscopes typically comprise a tube, which is inserted into the body, halving viewing or imaging capabilities and channels that are utilized for air insertion, water injection, suction and for passing medical devices through them into the body. The tube is connected, at its proximal end, to a control body that is held by an external operator. Feature buttons and pulley wheels are presented on the control body for activation and control of the endoscope, the different channel functions and the inserted medical devices. The design of medical devices used with endoscopes is subject to the endopscope limitations. The devices may have to be miniaturized in order to accommodate to the endoscope channel dimensions (for example, devices utilized in gastroenterology are typically passed through channels that measure 2mm to 4.2mm). Many surgical procedures cannot be effectively conducted with these miniaturized surgical instruments. Thus, the greatest limitation for gastrointestinal surgery today is the limited access through small endoscope channels.

In vivo procedures are sometimes required for providing emergency aid in the field, such as, at the location of an accident. These in vivo procedures may include, among other things, suction and intubation. Suction may be performed, for example, in cases of acute gastric bleeding or stomach emptying, for treating acute poisoning etc. Intubation may be performed, inter alia, to facilitate pulmonary ventilation during anesthesia or in intensive care situations. The known devices or systems for performing in vivo procedures are usually bulky and may have to be connected to an external power supply, or piping system. Furthermore, the known devices usually have to be sterilized in between procedures. Thus, the known devices cannot realistically accommodate the patient and/or medical needs during emergency in vivo procedures, in which a power supply and piping system, as well as sterilization, might not be easily available and in which transport and/or movement of the patient may be required.

### SUMMARY OF THE INVENTION

There is thus provided, according to an embodiment of the invention, a tool and system for performing in vivo procedures. The tool and system, according to an embodiment of the invention, is not subject to endoscope limitations since the device is usually self-contained, combining in vivo performing capabilities in a single integrated device. The tool and system according to an embodiment of the invention, may be a single-use device or may comprise single-use components, essentially eliminating the need for sterilization in between uses. According to an embodiment of the invention, the tool and system may be capable of performing in vivo procedures without wired connections, or with reduced numbers of wired connections, to external apparatus. Thus, the tool and system according to an embodiment of the invention can be easily used in emergency in vivo procedures.

The term "in vivo procedures" relates to any diagnostic and/or therapeutic procedures performed in the human body, for example, but not limited to, in vivo sensing, in vivo imaging, procedures of gastroenterology, procedures within blood vessels, procedures of gynecology and laparoscopic surgery procedures. The invention is as defined in the appended set of claims.

There is thus provided, according to one embodiment of the invention, an insertion member having a proximal end, which is accessible to an external operator and a distal end, which is inserted in vivo. The insertion member, according to an embodiment of the invention, comprises, at its distal end an imaging unit. The imaging unit, according to an embodiment of the invention, comprises a complementary metal oxide semiconductor (CMOS) imaging chip, an illumination source, such as a light emitting diode (LED), optic fibers or a luminescent foil, and a transmitter for transmitting image data from the image sensor to a typically external receiving system. According to an embodiment of the invention, the image sensor and illumination source are situated behind a single optical window. Optionally, some components of the imaging unit may be battery operated, while others, may be connected through a wired connection to an external power supply. The insertion member, according to an embodiment of the invention, may be a single-use member or comprise some parts that are single-use, such as a single-use imaging unit.

There is also provided, according to another embodiment of the invention, a device for performing an in vivo procedure. According to an embodiment of the invention, the device comprises a central body having a distal end, which is inserted in vivo, and a proximal end, which is accessible to an external operator. The device comprises at its distal end, a functional unit and an imaging unit. The functional unit includes at least one instrument for performing an in vivo procedure. The imaging unit comprises at least one illumination source for illuminating a site in vivo, at least one image sensor for obtaining images of the site in vivo and an optical window. The imaging unit may also comprise a transmitter for transmitting image data from the image sensor to a receiving system, typically located externally to a patient's body. At its proximal end, the device comprises controls that are functionally or electrically coupled to the functional unit for externally activating and manipulating the functional unit for performing in vivo procedures. The imaging unit may be a physically distinct unit located at the distal end of the device. Alternatively, the imaging unit components may be each positioned, on the distal end of the device, in accordance with specific requirements of the functional unit, of the specific site in vivo, of illumination conditions etc. The components of the imaging unit, which may be as described above, may be powered by wires connected to an external power source. The wires may run through or along the central body of the device. Alternatively, the components of the imaging unit may be wireless, utilizing a contained energy source, such as a battery.

There is further provided, in accordance with another embodiment of the invention, a multi-piece endoscope having a preferably re-usable hand piece section and a preferably single use insert section. In use, the insert section is inserted into the body, while the hand piece section allows the medical professional to control and interface with the endoscope. In another embodiment, the invention comprises an essentially wireless, self-contained endoscope capable of performing all endoscopic functions without wired connections, or with reduced numbers or wired connections, to external apparatus, such as monitors/video processors, or a power source.

Also provided, in accordance with claim 13 of the invention, is a system for performing in vivo procedures. According to one embodiment, the system comprises a tool having an in vivo sensor for obtaining in vivo information and for performing an in vivo procedure and a receiver, processor and monitor in communication with the tool for receiving and optionally processing the in vivo information obtained by the tool and for optionally displaying the in vivo information. In one embodiment the tool comprises an image sensor for obtaining in vivo images. The tool may further comprise a transmitter for transmitting data, such as image data, from the in vivo sensor to the processor. The data may then be displayed on the monitor. The monitor may be, inter alia, a computer or video monitor or a specifically designed LCD. The tool, may be a single-use, self-contained tool. Preferably, the communication between the elements of the system may be wireless. Also, the elements of the system are preferably portable. Thus, the system, according to claim 13 of the invention, can be easily used in emergency cases or in the field.

### BRIEF DESCRIPTION OF THE DRAWINGS

The present invention will be understood and appreciated more fully from the following detailed description taken in conjunction with the drawings in which:

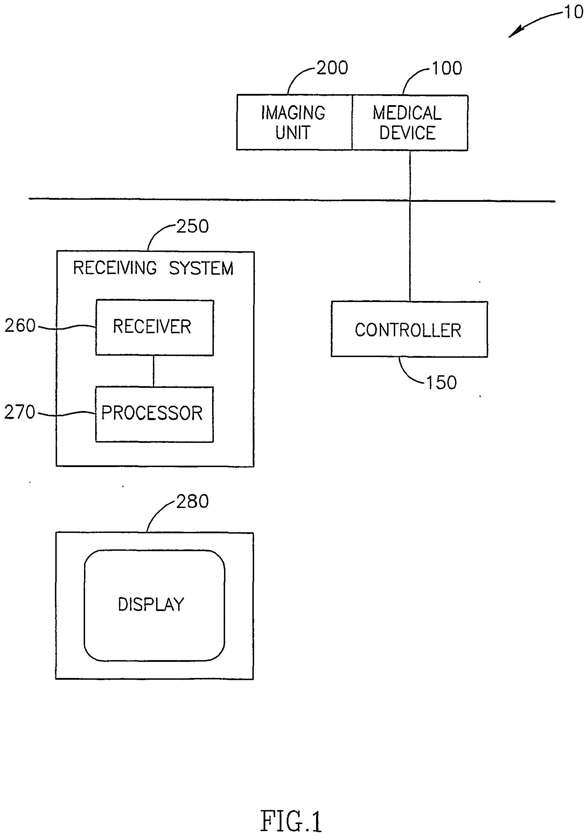

Figure 1 is a block diagram of a system in accordance with an embodiment of the invention;

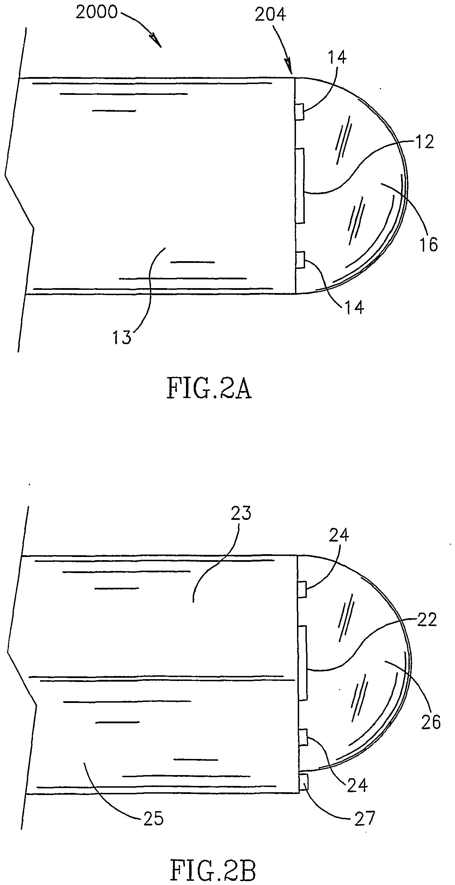

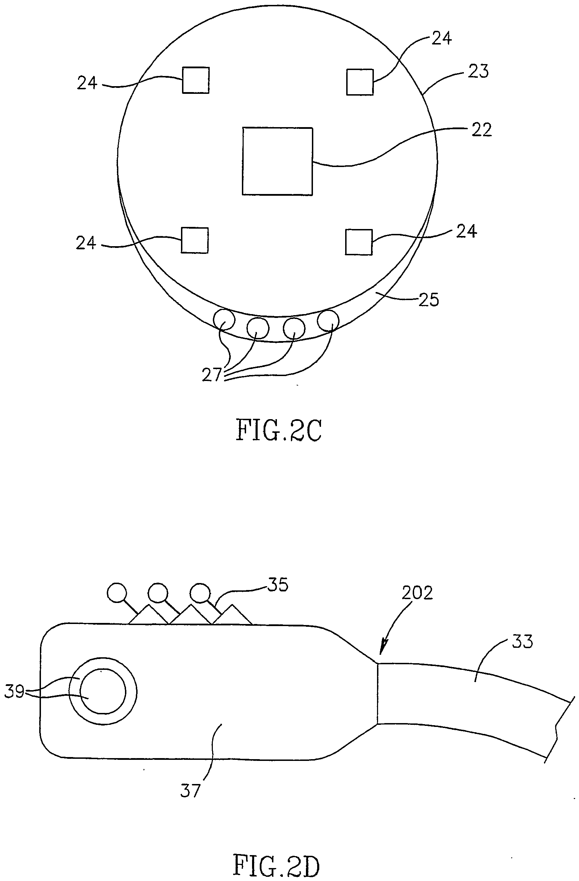

Figures 2A-D are schematic illustrations of a device in accordance with an embodiment of the invention; Fig. 2A is a schematic longitudinal cross section illustration of the device in accordance with an embodiment of the invention; Fig. 2B is a schematic longitudinal cross section view illustration of the device with added work channels in accordance with an embodiment of the invention; Fig. 2C is a schematic radial cross section view of the device in Fig. 2B; and Fig. 2D is a schematic side view illustration of a control body in accordance with an embodiment of the invention;

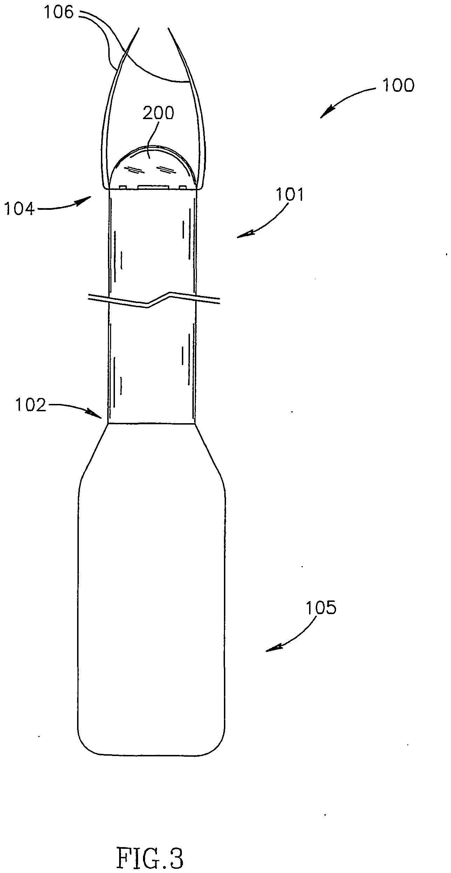

Figure 3 is a schematic side view illustration of tool according to an embodiment of the invention; and

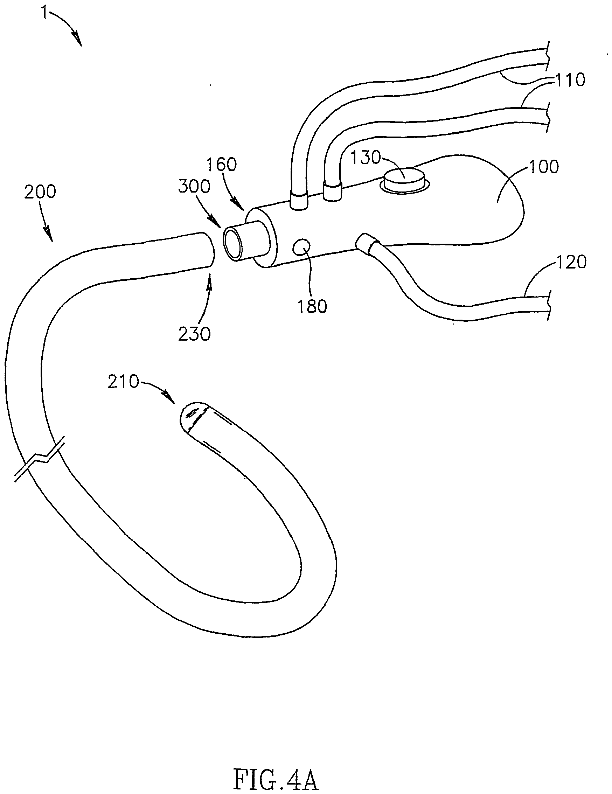

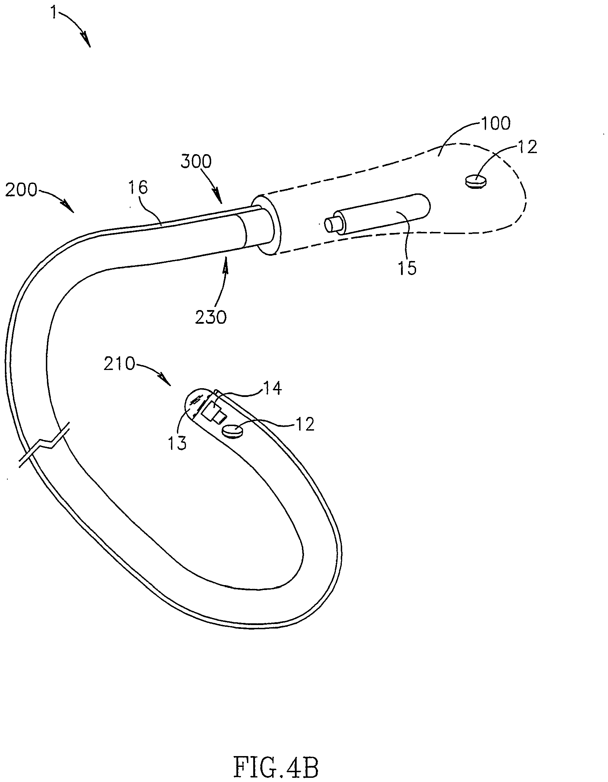

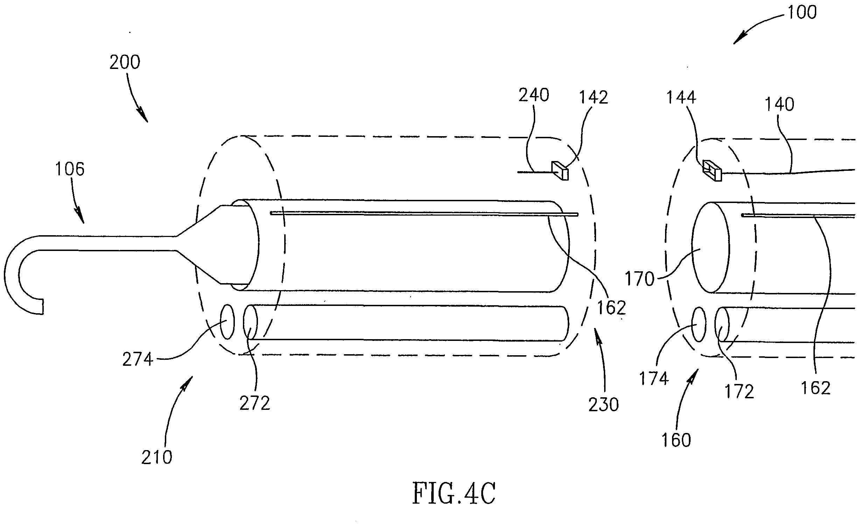

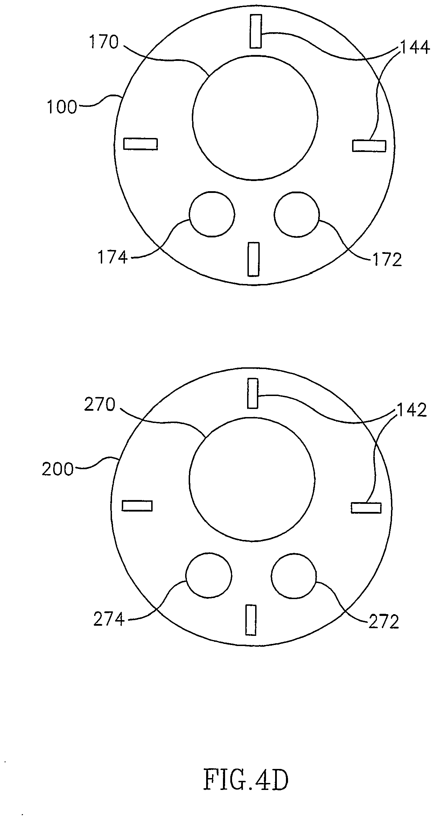

Figs. 4A-D and 4B are schematic illustrations of a multi-piece endoscopr in accordance with a n embodiment of the invention; Fig. 4A is a schematic illustration of a two-piece endoscope according to an embodiment of the invention; Fig. 4B is a schematic illustration of a two-piece endoscope according to another embodiment of the invention; Fig. 4C depicts the interface between a central body and controller according to one embodiment of the present invention; and Fig. 4D is a cross section view of the interface between a central body and controller according to one embodiment of the present invention.

### DETAILED DESCRIPTION OF THE INVENTION

Embodiments of the present invention relate to a system for performing in vivo procedures in human or animal patients, for example, procedures of gastroenterology, procedures within blood vessels, procedures of gynecology, laparoscopic surgery procedures and so on. In preferred embodiments, the system contains portable and single-use components which can operate essentially wirelessly, rendering the system essentially self sufficient and easily operable in the field or in emergency situations.

The device according to embodiments of the invention is typically an integrated device combining capabilities, such as visualization and performance capabilities. Further, in accordance with an embodiment of the invention, the device has autonomous imaging capabilities and can operate in vivo independently of guiding apparatuses, such as endoscopic instruments. The device, which is designed according to functional considerations and not according to endoscope limitations, can cover a wider range of in vivo procedures than medical devices that are endoscope dependent.

Devices for performing in vivo procedures, according to an embodiment of the invention may include, but are not limited to, graspers, blades, clamps, tissue collecting baskets, means for delivering treatment at a specific location, stents, catheters, suturing devices, forceps, dilatation balloons etc.

In the following description, various aspects of the present invention will be described For purposes of explanation, specific configurations and details are set forth in order to provide a thorough understanding of the present invention. However, it will also be apparent to one skilled in the art that the present invention may be practiced without the specific details presented herein. Furthermore, well known features may be omitted or simplified in order not to obscure the present invention.

Reference is now made to Figure 1, which is a block diagram of a system for performing in vivo procedures, in accordance with an embodiment of the invention. In an exemplary embodiment, the system 10 preferably includes parts that operate inside the patient's body (in vivo) and parts that operate outside the body. The in vivo parts are preferably parts intended for single use, such as an imaging unit 200 and a medical device 100. The single use parts can be replaced between uses, thus eliminating the need to sterilize the parts after every use. The parts that operate outside the body are preferably reusable, for example, a controller 150, typically for controlling the medical device 100, a receiving system 250, which typically comprises a receiver 260 and a processor 270, for receiving and optionally processing image data from the imaging unit 200 and a display 280 for displaying the image data received from the imaging unit 200. System 10 may be constructed as a single device including all the components of the system. Other configurations are possible. For example, the receiving system 250 may be remotely positioned and in wireless communication with the imaging unit 200 and/or the medical device 100. The display 280, which may be a computer or video monitor or a specifically designed LCD, may be part of the receiving system 250 or may be a separate unit, connected by wire to or in wireless communication with the receiving system 250.

Preferably, the controller 150 is in communication with the medical device 100 so as to control its operation. The imaging unit 200 is preferably attached to the medical device 100 but may also be a separate unit. The imaging unit 200 transmits image information to the receiving system 250, preferably to the receiver 260, over a wired connection or wirelessly. The receiver 260 and the processor 270 may be in communication with each other and processor 270 may process the image data received by the receiver 260.

According to one embodiment, real-time viewing of a body lumen is enabled by the system 10. Alternatively, the receiving system 250 may include a memory (not shown), optionally a portable memory unit such as a CD, for saving the in vivo data transmitter from the imaging unit 200 for later display and/or analysis of the data.

The receiver 260 and processor 270 may be constructed as a one-part unit wherein the receiver is incorporated in the processor. In another embodiment, the receiver 260, processor 270 and display 280 may all be part of a single unit. In yet another embodiment, the receiver 260 is a separate unit and the processor 270 and display 280 comprise a separate unit. The receiver 260, which may include a memory or recording mechanism (not shown), may be portable and carried in proximity to a patient's body while the patient is being viewed by imaging unit 200. Image data is transmitted from the imaging unit 200 to the receiver 260 and is saved or recorded onto the memory in the receiver 260. The saved or recorded data can then be downloaded or otherwise transferred to the processor 270 for analysis and/or further display on the display 280. A receiving system, including a display which can be implemented in an embodiment of the invention is described in U.S. Patent 5,604,531 to Iddan, which is assigned to the common assignee of the present invention. Also, the RAPID^{™} work station by Given Imaging Ltd. Of Yokneam, Israel, which includes a receiver, processor and display, may be easily modified by a person skilled in the art to be operable in the present invention. Other receivers and/or processors and/or displays may be used. Also, in alternate embodiments, the system 10 may include other components in other arrangements. Additionally, other methods may be used to transmit images from the imaging unit to the receiver system.

A schematic illustration of a device, in accordance with an embodiment of the invention, is presented in Figs. 2A-D. In Fig. 2A a device 2000 includes an insertion member 13 which comprises, at its distal end 204, a CMOS imager 12, light emitting diodes (LEDs) 14 and an optical window 16. The insertion member 13 may be designed to meet specific requirements. Insertion member 13 may be a flexible or rigid rod or it may be specifically shaped. Insertion member 13 may be made of any suitable material such as silicon, suitable plastics, suitable metals, etc. The CMOS imager 12 may be an active or passive CMOS imaging chip and may generate digital or analog signals. Preferably, CMOS imager 12 is a single chip imager similar to the CMOS image sensor (Camera on Chip) designed by Photobit Inc. of California, USA, with integrated active pixel and post pixel circuitry. LEDs 14 may be monchromatic or white LEDs. A CMOS image sensor and LED operable in accordance with an embodiment of the invention are described in WO 01/65995, which is assigned to the common assignee of the present invention. CMOS imager 12, LEDs 14 and possibly lenses or mirrors for collimating remitted light (not shown), are positioned behind optical window 16. In the embodiment illustrated in Figs. 2A and 2B optical window 16 is dome shaped.

For optimizing imaging conditions optical window 16 can be configured to define an ellipsoid shape and the CMOS imager 12 and LEDs 14 can be positioned on the focal plane of the shape defined by the optical dome, as described in WO 00/76391, which is assigned to the common assignee of the present invention.

The components of the insertion member 13 may be powered through wires connecting them with an external power source (not shown). Alternatively, it will be appreciated that both CMOS imagers and LEDs are low power components that may be powered by a battery (not shown).

Transmission of signals from the CMOS imager 12 may be effected through wires connecting the CMOS imager to a remote and external receiving system (not shown). Alternatively, a wireless transmitter may be utilized for transmitting signals to the receiving system. Signals from CMOS imager 12 may be transmitted using various digital or analog modulation techniques. For example, transmission of a digital image over a radio channel may use an FSK (Frequency Shift Keying) modulation technique. Preferably, the transmitter wirelessly transmits image data to an external receiving system, for example, by using microwave or radio frequencies. In one embodiment, the imaging unit is a single-use, battery-operated unit. In other embodiments the imaging unit may be externally induced, such as by an external magnetic field, or may be connected to an external power supply. Optionally, some components of the imaging unit may be battery operated, such as the image sensor and illumination source, while others, such as the transmitter, may be connected through a wired connection to an external power supply. It will be appreciated that the wireless embodiment has the advantage of being more easily disposable and less cumbersome than the wired embodiment.

Device 2000 may serve as an accessory to utility devices for in vivo diagnostics and/or therapeutics. Alternatively, device 2000 may serve as a platform for adding integrated utility devices for in vivo diagnostics and/or therapeutics.

An embodiment which includes additional utility devices is illustrated in Figs. 2B and 2C. The embodiment illustrated in Figs. 2B (a longitudinal cross section view illustration) and 2C (a radial cross section view) includes insertion member 23 which comprises CMOS imager 22, LEDs 24 and optical window 26. Further included is an area 25, which forms a space or matrix enclosing insertion member 23 and through which channels 27 traverse. Channels 27 may be air or water channels or they may be suction channels or channels for receiving utility devices. Utility devices may include, but are not limited to, graspers, blades, clamps, tissue collecting baskets, means for delivering treatment at a specific location, stents, catheters, suturing devices, forceps, dilatation balloons etc..

Utility devices for in vivo diagnostics and/or therapeutics, which are inserted through channels 27, may be controlled by a control body, that is connected to the insertion member.

A control body, in accordance with an embodiment of the invention, is illustrated in Fig. 2D. Insertion member 33 is connected at its proximal end 202 to control body 37. Control body 37 comprises device controls 35 for manually manipulating the utility devices and control knobs 39 for moving the distal end of insertion member 33. Utility devices that are inserted through channels 27 are coupled at their proximal end to device controls 35 such that the utility device distal end, which is inserted in vivo (for example, in a patient's body), can be manipulated by moving device controls 35. The insertion member distal tip and the utility devices may also be controlled mechanically or automatically, as known in the art.

A medical tool according to an embodiment of the invention is illustrated in Fig. 3. The medical tool 100 comprises a central body 101 having a distal end 102 and a proximal end 104. The proximal end 104 contains a functional element, such as, blades 106, and an imaging unit 200. A controller 150 for controlling the action of the functional element, such as blades 106, is attached at the distal end 102 of the central body 101. The controller 150 may be handles or any other suitable controlling element that is functionally coupled to the blades 106 through the central body 101. Alternatively, the controller 150 may be an electronic command box electrically coupled to the functional unit either wirelessly, by using IR, radio waves etc., or through the central body 101, for example by wires running through the central body 101 and connecting the blades 106 with the controller 150. Controller 150 is preferably accessible to an external operator to be operated manually by applying force so as to control and manipulate the blades 106. In one embodiment controller 150 may be connected to an external electric power supply or to a battery so as to allow electric operation of the medical tool 100. Alternatively, other methods of controlling blade movements may be used.

The central body 101 can be adjusted to be employed in any in vivo procedure. It can be flexible (for example, to be used in procedures of gastroenterology), rigid or semi rigid, as required. It can be fabricated of any suitable material such as silicon, suitable plastics, suitable metals etc.

Imaging unit 200, is located at the distal end 104 of the central body 101, such that the operation of blades 106 and the in vivo site of operation can be imaged and viewed simultaneously with the real time operation of blades 106. Imaging unit 200 may be similar to the imaging unit described in Fig. 1. Images can be transmitted from imaging unit 200 to a receiving unit as described in Fig. 1 and can be viewed in real time or stored in a receiver for later viewing.

It will be appreciated that although a specific functional unit for performing an in vivo procedure (blades) is demonstrated, the device of the invention is not limited to these components. Rather, the functional units for performing an in vivo procedure may include graspers, blades, clamps, tissue collecting baskets, means for delivering treatment at a specific location, stents, forceps, etc. More specifically the device may utilize instruments from these exemplary categories: biopsy forceps, polypectomy snares, hemostasis devices (elctro-cautery, band ligation, endoclips), dilatation balloons, catheters, sphincterotomes, guidwires and suturing devices. The device 100 may be attractive to apply on advanced surgical devices such as hemostasis cautery and band ligation devices, gastrointestinal resection devices, gastro fundo-plication devices and gastrointestinal suturing and clipping devices.

Another exemplary embodiment of the system of the present invention is illustrated in Figs. 4A-C. According to an embodiment of the invention, the system includes a multi-piece endoscope comprising a preferably re-usable hand piece section and a preferably single use insert section. In use, the insert section is inserted into the body of a human or animal patient (e.g., the GI tract, circulatory system, abdomen, or other cavity or lumen). The hand piece section remains partially or completely outside the body, and provides the interface and controls (*e.g*., pulleys, air/water controls, suction controls), which the medical technician (*e.g*., the gastroenterologist, surgeon, etc.) operates, and optionally provides the interface to external supplies, monitors, or other equipment (*e.g*., air, water, a video monitor). Preferably, different types of insert sections may be used with the same hand piece section, including insert sections having varied functionalities, uses, and structures.

In an exemplary embodiment, the hand piece section and the insert section connect at an interface. Preferably, the interface performs several tasks. For example, the interface, *inter alia,* physically connects the hand piece section and the insert section, allows physical control information, such as mechanical force provided by pulleys, to pass from one section to the other, allows power (*e.g*., electrical power) to pass between the sections, and allows other information (such as video signals or fiber optic information) to pass between the sections. The interface may provide a seal for tubes running through both sections which allow physical matter (*e.g*., air, water) to pass between the sections, and may connect tubes running through both sections through which instrument inserts such as graspers, blades, clamps, tissue collecting baskets, means for delivering treatment at a specific location, stents, catheters, suturing devices, forceps, dilatation balloons etc., are inserted into the body.

Reference is made to Fig. 4A, which depicts a two-piece endoscope according to one embodiment of the present invention. In an exemplary embodiment, the two-piece endoscope 1 comprises a hand piece section 100 having a distal end 160, and an insert section 200 having a distal tip 210 and a proximal end 230; the hand piece section 100 and an insert section 200 are connected at an interface 300. The hand piece section 100 may include tubes 110 for air and water, wires 120 delivering, for example, electric power to the endoscope 1 or signals to a monitor or computer (not shown). Tubes 110 may allow for, for example, insufflation, suction, or flushing. The hand piece section 100 may include, for example, a set of controls 130, such as controls for moving the distal tip 210 of the insert section 200. The controls 130 may act to control, for example, air, water, suction, insuflation and/or flushing. The hand piece section 100 may include an opening 180 for inserting an instrument.

In another embodiment depicted in Fig. 4B, the endoscope 1 is an essentially autonomous endoscope, minimally or not at all relying on connections to external apparatuses, thereby enhancing mobility and easy use of the endoscope 1. The endoscope 1 includes at the distal tip 210 of the insert section 200, an imaging unit 13 such as the imaging unit described above or in WO 00/76391 or the imaging system described in US Patent Number 5,604,531 or WO 01/165995 all of which are assigned to the common assignee of the present invention. The imaging unit 13 may include an image sensor, such as a CCD or a CMOS image sensor, an optical system (which typically includes lenses and/or mirrors and/or prisms) and an illumination source, such as LEDs or optical fibers. The image information from the imaging unit may be transmitted to the hand piece section 100 by, for example, a wire. Alternately, the image information may be transmitted without a wire; for example using a radio transmitter to a receiving unit located in the to the hand piece section 100 or in an alternate location.

If the images are sent to the to the hand piece section 100 (by wire or by radio waves), the hand piece section 100 may send the image information to a monitor, recorder, data processor, or other device. The hand piece section 100 may transmit such information by wire or by wireless transmission. For example, the hand piece section 100 may include a transmitter 14, which may be, for example, an RF transmitter such as the transmitter described in the above mentioned WO01/65995. Alternately, the imaging unit 13 may include a transmitter and transmit image or other information directly to a monitor, recorder, data processor, or other device. All or some of the elements of the imaging unit 13 may be powered by a battery 12, which may be a single use or a rechargeable battery, contained at the distal tip 210. Alternatively the elements may be powered by a battery 12 located elsewhere along the endoscope 1, for example, in the hand piece section 100.

In one embodiment, the insert section 200 may include a water reservoir 16. For example, a 130cm endoscope with 13mm diameter has a gross volume of 172cc. A working channel of 3.5mm has a volume of 12.5cc, and two water/air channels, of 1mm diameter, each have a volume of 4cc. Therefore, the net volume of such an endoscope is approximately 150cc (172 - 12.5 - 4 - 4) = 151.5). This volume of water may be used for flushing the lumen. In alternate embodiments an endoscope containing a water reservoir may have different configurations, and may include a water reservoir having different configurations.

In one embodiment, a compressed air/gas balloon 15, built inside the hand piece section 100, provides air pressure to insufflate or flush water. Alternatively or additionally, a small electrical pump can be incorporated in the hand piece section 100, to provide pressure and/or suction, or charge pressed air into the balloon 15. The content sucked out of the body lumen can be deposited in the emptied space in the water reservoir 16. In alternate embodiments an endoscope containing a gas or air balloon or reservoir may have different configurations, and may include a gas or air balloon or reservoir having different configurations.

It will be appreciated that the endoscope in its wireless embodiment (for example as illustrated in Fig. 4B) may be a single piece endoscope or any endoscope designed for single use or may be a two-piece endoscope, for example, as illustrated in Fig. 4A.

Reference is made to Figs. 4C and 4D, which depict the interface of a two-piece endoscope according to one embodiment of the present invention. Referring to Figs. 4A, 4C and 4D, the distal end 160 of the hand piece section 100 and the proximal end 230 of the insert section 200 are connected at an interface 300. Wires (not shown) controlling the distal tip 210 of the insert section 200 are disposed in the hand piece section 100, and are controlled by controls 130 in a known manner. Preferably four wires are included; other numbers of wires may be used. Preferably, the controls 130 include two pulleys, a horizontal pulley and a vertical pulley, and, for each pulley, when the pulley is moved in one direction, one of the two wires is pulled towards the controls 130, and one of the two wires is released away from the controls 130. Other methods of moving the wires and of translating wire movements to endoscope movements may be used.

Wires (not shown) controlling the distal tip 210 of the insert section 200 are disposed in insert section 200. These wires act to move the distal tip 210 of the insert section 200 in a known manner.

When the hand piece section 100 and insert section 200 are connected, the wires disposed in the hand piece section are attached to the wires disposed in the insert section, and thus the control information (such as mechanical force) transmitted by the wires disposed in the hand piece section is transmitted by the wires disposed in insert section to the distal tip 210. The wires may be connected in various manners. In one embodiment, each of the wires disposed in the hand piece section includes a preferably rigid loop and each of the wires disposed in the insert section includes a hook; when the hand piece section 100 and insert section 200 are properly connected, the hooks enter the loops and thus connect the wires. When the proximal end 230 of the insert section 200 is inserted to the distal end 160 of the hand piece section 100, the hand piece section 100 and insert section 200 are rotated in opposite directions, placing the hooks inside the loops. Once the hooks are inside the loops, the wires disposed in the hand piece section may be retracted mechanically away from the wires disposed in the insert section to create tension on the wires, decreasing slack and increasing control. In one embodiment, the mechanical retraction is achieved by a knob located, for example, at the distal end 160 of the hand piece section 100, which is connected to a set of threaded members located at the origin of the wires disposed in the hand piece section. Rotating the knob rotates the set of threaded members, pulling the wires disposed in the hand piece section away from the distal end 160, creating tension. The bases of the wires disposed in the hand piece section may contain threads corresponding to the set of threaded members; other configurations are possible. Once connected, the wires disposed in the hand piece section pull on the wires disposed in the insert section to control the distal tip 210. In alternate embodiments, such a retraction mechanism may have other configurations; for example, control knobs for the mechanism may be placed with the set of controls 130. In alternate embodiments the wires disposed in the insert section may have loops, and the wires disposed in the hand piece section may include hooks.

Other methods of connecting the wires may be used, and alternate methods of connecting the hand piece section 100 and insert section 200 may be used. For example, each of the wires disposed in the hand piece section may include a spring at its origin in the hand piece section 100. Each spring applies a force pulling the corresponding wire disposed in the hand piece section away from the distal end 160 of the hand piece section 100. The hooks on the ends of wires disposed in the insert section have a preferably triangular profile. When the hooks are inserted through the loops on the wires disposed in the hand piece section, the mechanical force of the angled triangle hooks pull the loops towards the distal end 160 of the hand piece section 100, extending the springs. Tension is thus created. Preferably, at the corner of the triangle a small niche is included which allows the triangle to fit into the loop tightly and locks the triangle; a "click" sound may announce the proper placement. Other methods of attaching the wires, and, possibly, creating tension on the wires, may be used. In further embodiments a tension inducing mechanism need not be used.

In further embodiments, the use of wires to control distal tip movement may not be used; other methods may be used, or, alternately, no movement controls need be included. For example, an insert section used for intubating may not require movement controls. Furthermore, given that, preferably, various types of insert sections may be connected to the hand piece section, an insert section not including wire controls may be attached to a hand piece section including such controls.

Preferably, the hand piece section 100 and insert section 200 each include cavities or channels for, for example, water, air, suction, and instrument insertion. For example, hand piece section 100 includes instrument channel 170, air channel 172, and water channel 174. Preferably, insert section 200 includes channels or cavities that, when the hand piece section 100 and insert section 200 are connected, match, so that materials or fluids (e.g., air or water) or instruments may travel through both the hand piece section 100 and insert section 200 uninterrupted and without leakage. Thus, in one embodiment, insert section 200 includes instrument channel 270, air channel 272, and water channel 274 which, when the hand piece section 100 and insert section 200 are properly connected, are connected to and positioned opposite instrument channel 170, air channel 172, and water channel 174, respectively.

Preferably, at the point the channels or cavities meet at the interface 300 (at the proximal end 230 and the distal end 160), seals such as rubber, silicon or plastic seals or washers ensure that, when the insert section 200 and hand piece 100 are properly connected, the channels or cavities are properly sealed to one another. Certain channels or cavities may not require such seals, and other methods of ensuring a seal may be used.

In alternate embodiments, other sets of channels or cavities may be included in the hand piece section and insert section. In alternate embodiments, the cavities in each of the hand piece section 100 and insert section 200 need not match, given that, preferably, various types of insert sections may be connected to the hand piece.

In one embodiment, the insert section includes an imager, for example a CMOS imager, for viewing the inside of the body and possibly a light source, such as an LED light source. Electrical connections for, for example, power, images, and controls, may be provided by wires extending through the hand piece section 100 and insert section 200 and having contacts at the interface 300. When the hand piece section 100 and insert section 200 are properly connected, the contacts for the wires on the hand piece section 100 match to the contacts for the wires on the insert section 200, establishing an electrical connection. In alternate embodiments, other instruments requiring electric power or electronic information may be included. Other types of connections may be made between hand piece section 100 and insert section 200; for example, a fiber optic connection allowing for viewing of body cavities.

In one embodiment, the interface 300 includes a connection system such as matching screw type threads or a coaxial connection. For example, to connect the hand piece section 100 and insert section 200, the user first connects the wires 140 and wires 240, and possibly other connections. The user then inserts the hand piece section 100 into the insert section 200 and turns. As the user turns, the hand piece section 100 and insert section 200 are pressed against one another. In one embodiment, the hand piece section 100 includes an outer ring that includes an internal engraved thread. The insert section 200 includes flaps or extensions fitting into the threads. When the ring is placed over the flaps and begins to rotate, the flaps and the insert section 200 are pulled towards the hand piece section 100, so as to press them firmly against each other. Preferably, any electrical contacts in the interface 300 are also pressed against one another, and any seals for channels or cavities are also pressed against one another. In alternate embodiments, other systems for connecting the hand piece section 100 and insert section 200 may be used; for example, a clamp system, or a system where the insert section 200 includes threads. Alternatively, The hand piece section 100 and insert section 200 may be connected in one motion.

In another embodiment, which can be useful in an autonomous endoscope, according to an embodiment of the invention (for example as illustrated in Fig. 4B), pressurized air is forced from the hand piece section 100 into the insert section 200 either to insufflate a body lumen or to drive water from an internal reservoir into the body lumen. In this embodiment a nozzle is mechanically/manually emerged from the hand piece section 100 and entered into an air channel, after the both are connected by interface 300. The nozzle is inserted into an air channel in the insert section 200 and seals the rim of the air channel opening once inside, thus preventing leakage of air when pressurized air is released into the air channel. The pressurized air can be supplied from an external source or from an internal source, such as the balloon 15 demonstrated in Fig. 4B.

A further embodiment an autonomous endoscope according to an embodiment of the invention (for example as illustrated in Fig. 4B) includes an air/water selector. In this embodiment selection of the "flush" control, for example, by an operator will cause pressurized air to be directed into the water reservoir (such as water reservoir 16 in Fig. 4B), in order to force water out of the distal tip 210. A "router" of pressurized air is placed toward the proximal end 230 of the insert piece 200 to direct the air pressure into the water reservoir when "flush" is selected The router may be placed, for example, immediately after the nozzle referred to above.

Preferably, the hand piece section 100 is intended to be used repeatedly, and is manufactured accordingly, and preferably the insert section 200 is intended for single use, and is also manufactured accordingly. Preferably, the materials required to be so are biocompatible. In alternate embodiments, the insert section 200 need not be single use.

Preferably, various insert sections, having different functionalities, structures and uses, may be attached to and used with the same hand piece section. In alternate embodiments, a hand piece section may be intended for use with only one type of insert section. In further embodiments, the endoscope need not be "two-piece," and an endoscope according to the present invention may include multiple sections.

It should be appreciated that any of the embodiments described above or other embodiments, according to the invention, can be combined to form a system in accordance with an embodiment of the invention. The system would have the benefit of performing medical procedures in the field or in emergency cases while obtaining in vivo information of the patient, typically, in vivo image information. This may be particularly intended for cases of internal bleeding where it might be crucial to view and locate the in-vivo bleeding organ in order to perform a successful treatment and for cases of obstructed airway where a medical tool such as an intubation tool may be inserted. It will be appreciate that the device is not limited for use in case of bleeding or obstructed airway but can be use in other treatments as well.

For example, a system for emergency treatment in the field may include a medical tool comprising an imaging unit capable of transmitting image data and a receiving unit. The receiving unit, which comprises a receiver, processor and a display may be part of a portable computer (e.g., a PC or palm-top computer) and will allow the processing of in vivo information and the viewing of the in-vivo images without requiring a connection to an external power supply.

In alternative embodiments a medical tool may comprise other in vivo sensors, such as known in vivo pH meters, in vivo pressure detectors, temperature sensors, etc. The in vivo sensors may transmit in vivo data to an external receiving unit as described above.

Also, a single-use, multi-piece endoscope according to an embodiment of the invention may be included in a system with a receiving unit as described above. In another embodiment a small electrical pump can be incorporated in the endoscope hand piece or in communication with the hand piece to provide pressure and/or suction, or charge pressed air for flushing a body lumen.

The system and device according to an embodiment of the invention may be used in a portable emergency kit, such as an "emergency suitcase". The "emergency suitcase", according to an embodiment of the invention contains a single-use, self contained, wirelessly operated device for performing in vivo procedures with integrated visualization and a portable unit comprising a receiver, processing unit and display. Utilizing the "emergency suitcase" in accordance with an embodiment of the invention will enable performing emergency in vivo procedures in the field.

It will be appreciated by persons skilled in the art that the present invention is not limited to what has been particularly shown and described hereinabove. Alternate embodiments are contemplated which fall within the scope of the invention.

## Claims

1. A medical tool (100) comprising a central body (101) having an in vivo inserted end (104) and a non inserted end (102),
**characterized in that** at least one functional unit (106) for performing an in vivo procedure is integrated in said central body (101) at said in vivo inserted end (104);
and **in that** at least one imaging unit (200) is integrated in said
central body (101) at said in vivo inserted end (104), **in that** said imaging unit (200) comprises a dome shaped optical window (16), an image sensor (12), a lens and an illumination source (14), and
**in that** the image sensor (12), the lens and the illumination source (14) are positioned behind and operate through the dome shaped optical window (16).

2. The tool according to claim 1 wherein the non inserted end (102) includes a controller (150), said controller (150) functionally coupled to the functional unit (106).

3. The tool according to claim 1, said tool including silicon, plastic or metal.

4. The tool according to claim 1 wherein the functional unit (106) comprises one or more element selected from the group consisting of: graspers, blades, clamps, tissue collecting baskets, means for delivering treatment, stents, forceps, snares, hemostasis devices, dilatation balloons, catheters; sphincterotomes, guidwires and suturing devices.

5. The tool according to claim 1 wherein the illumination source (14) is a LED.

6. The tool according to claim 1 comprising a channel (27), wherein said channel (27) passes through at least the central body (101).

7. The tool according to claim 1, wherein at least said functional unit (106) and said imaging unit (200) are for single use.

8. The tool according to claim 1, wherein said imaging unit (200) comprises an optical system positioned behind the dome shaped optical window (16).

9. The tool according to claim 1, wherein said tool comprises a battery (12).

10. The tool according to claim 1, comprising a transmitter (14).

11. The tool according to claim 10 wherein the transmitter (14) is a wireless transmitter.

12. The tool according to claim 10 wherein the transmitter is an RF transmitter.

13. A system (10) for performing in vivo procedures, said system comprising: the tool according to claim 10, and a receiver (260) comprising a recording mechanism.

14. The system according to claim 13 comprising a monitor in communication with the receiver for displaying in vivo information.

15. The system according to claim 13 comprising a processor for processing in vivo information.

## Patentansprüche

1. Medizinisches Instrument (100) umfassend einen Zentralkörper (101) mit einem in-vivo eingeführten Ende (104) und einem nicht eingeführten Ende (102),
**dadurch gekennzeichnet, dass** zumindest eine Funktionseinheit (106) zum Durchführen eines in-vivo Eingriffs im genannten Zentralkörper (101) am genannten in-vivo eingeführten Ende (104) integriert ist;
und dass zumindest eine bildgebende Einheit (200) im genannten Zentralkörper (101) am genannten in-vivo eingeführten Ende (104) integriert ist, dass die genannte bildgebende Einheit (200) ein domförmiges optisches Fenster (16) umfasst, einen Bildsensor (12), Linsen und eine Beleuchtungsquelle (14), und
dass der Bildsensor (12), die Linsen und die Beleuchtungsquelle (14) hinter dem domförmigen optischen Fenster (16) angeordnet sind und durch dieses hindurch wirken.

2. Instrument gemäss Anspruch 1, wobei das nicht eingeführte Ende (102) einen Kontroller (150) umfasst, wobei der Kontroller (150) funktionell mit der Funktionseinheit (106) gekoppelt ist.

3. Instrument gemäss Anspruch 1, wobei das genannte Instrument Silikon, Plastik oder Metall umfasst.

4. Instrument gemäss Anspruch 1, wobei die Funktionseinheit (106) ein oder mehrere Elemente umfasst ausgewählt aus der Gruppe bestehend aus: Greifer, Messer, Klemmen, Gewebesammelkörbchen, Mittel zum Abgeben einer Heilbehandlung, Stents, Pinzetten, Schlingen, blutstillende Vorrichtungen, Dilatationsballone, Katheter, Sphincterotome, Führungsdrähte und Nahtversorgungsvorrichtungen.

5. Instrument gemäss Anspruch 1, wobei die Beleuchtungsquelle (14) eine Licht emittierende Diode ist.

6. Instrument gemäss Anspruch 1, umfassend einen Kanal (27), wobei der genannte Kanal (27) zumindest durch den Zentralkörper (101) verläuft.

7. Instrument gemäss Anspruch 1, wobei zumindest die genannte Funktionseinheit (106) und die genannte bildgebende Einheit (200) zur einmaligen Verwendung sind.

8. Instrument gemäss Anspruch 1, wobei die genannte bildgebende Einheit (200) ein hinter dem domförmigen optischen Fenster (16) angeordnetes optisches System umfasst.

9. Instrument gemäss Anspruch 1, wobei das genannte Instrument eine Batterie (12) umfasst.

10. Instrument gemäss Anspruch 1, umfassend einen Sender (14).

11. Instrument gemäss Anspruch 10, wobei der Sender (14) ein Drahtlossender ist.

12. Instrument gemäss Anspruch 10, wobei der Sender ein Radiofrequenzsender ist.

13. System zum Durchführen von in-vivo Verfahren, wobei das genannt System umfasst: das Instrument gemäss Anspruch 10, und einen Empfänger (260) umfassend einen Aufnahmemechanismus.

14. System gemäss Anspruch 13, umfassend einen in Verbindung mit dem Empfänger stehenden Monitor zum Anzeigen von in-vivo Information.

15. System gemäss Anspruch 13, umfassend einen Prozessor zum Verarbeiten von in-vivo Information.

## Revendications

1. Outil médical (100) comprenant un corps central (101) présentant une extrémité insérée *in vivo* (104) et une extrémité non insérée (102), **caractérisé en ce qu'**au moins une unité fonctionnelle (106) destinée à effectuer une procédure *in vivo* est intégrée dans ledit corps central (101) au niveau de ladite extrémité insérée *in vivo* (104) ;
et **en ce qu'**au moins une unité de formation d'image (200) est intégrée dans ledit corps central (101) au niveau de ladite extrémité insérée *in vivo* (104), **en ce que** ladite unité de formation d'image (200) comprend une fenêtre optique en forme de dôme (16), un capteur d'image (12), une lentille et une source d'illumination (14), et
**en ce que** le capteur d'image (12), la lentille et la source d'illumination (14) sont positionnés derrière la fenêtre optique en forme de dôme (16) et fonctionnent par l'intermédiaire de celle-ci.

2. Outil selon la revendication 1, dans lequel l'extrémité non insérée (102) comprend un dispositif de commande (150), ledit dispositif de commande (150) étant couplé de manière fonctionnelle à l'unité fonctionnelle (106).

3. Outil selon la revendication 1. ledit outil comprenant du silicium, une matière plastique ou du métal.

4. Outil selon la revendication 1, dans lequel l'unité fonctionnelle (106) comprend un ou plusieurs éléments sélectionnés à partir du groupe constitué de : éléments de préhension, lames, éléments de serrage, paniers de collecte de tissu, moyens destinés à délivrer un traitement, stents, forceps, pièges, dispositifs d'hémostase, ballons de dilatation, cathéters, sphinctérotomes, guides souples et dispositifs de suture.

5. Outil selon la revendication 1, dans lequel la source d'illumination (14) est une diode électroluminescente.

6. Outil selon la revendication 1, comprenant un canal (27), dans lequel ledit canal (27) traverse au moins le corps central (101).

7. Outil selon la revendication 1, dans lequel au moins ladite unité fonctionnelle (106) et ladite unité de formation d'image (200) sont destinées à un usage unique.

8. Outil selon la revendication 1, dans lequel ladite unité de formation d'image (200) comprend un système optique positionné derrière la fenêtre optique en forme de dôme (16).

9. Outil selon la revendication 1. dans lequel ledit outil comprend une pile (12).

10. Outil selon la revendication 1, comprenant un émetteur (14).

11. Outil selon la revendication 10, dans lequel l'émetteur (14) est un émetteur sans fil.

12. Outil selon la revendication 10. dans lequel l'émetteur est un émetteur haute fréquence.

13. Système (10) destiné à effectuer des procédures *in vivo,* ledit système comprenant: l'outil selon la revendication 10 et un récepteur (260) comprenant un mécanisme d'enregistrement.

14. Système selon la revendication 13, comprenant un écran en communication avec le récepteur en vue d'afficher des informations *in vivo.*

15. Système selon la revendication 13, comprenant un dispositif de traitement destiné à traiter des informations *in vivo.*
